# EUROPEAN PATENT SPECIFICATION

(11) **EP 1 185 216 B1**
(45) Date of publication and mention of the grant of the patent: **01.03.2006**
(21) Application number: 00937600.5
(22) Date of filing: 18.05.2000
(51) Int. Cl.: A61F 2/06

(54) **STENT-GRAFT WITH INCREASED FLEXIBILITY**
UMHÜLLTER STENT MIT ERHÖHTER FLEXIBILITÄT
STENT/GREFFE PLUS FLEXIBLE

(30) Priority: 20.05.1999 US 135031 P
(43) Date of publication of application: 13.03.2002
(73) Proprietor: Boston Scientific Limited, St. Michael (BB)
(72) Inventor: GOLDS, Ellen, Hastings-on-Hudson, NY 10706 (US); TSENG, David, Santa Rosa, CA 95405-8733 (US); BOATMAN, Jeff, Lincoln Park, NJ 07035 (US)
(74) Representative: Wittop Koning, Tom Hugo
(86) International application number: PCT/US2000/013665
(87) International publication number: WO 2000/071057

(56) References cited:
- EP-A- 0 689 805
- EP-A- 0 893 108
- WO-A-98/26731
- US-A- 5 549 663

## Description

The present invention relates generally to an implantable prosthesis used to repair or replace a body lumen. More particularly, the present invention relates to an endoluminal prosthesis including a stent and ePTFE graft composite device offering increased compliance and flexibility.

### BACKGROUND OF THE INVENTION

An endoluminal prosthesis is a medical device commonly known to be used in the treatment of diseased blood vessels. An endoluminal prosthesis is typically used to repair, replace, or otherwise correct a damaged blood vessel. An artery or vein may be diseased in a variety of different ways. The prosthesis may therefore be used to prevent or treat a wide variety of defects such as stenosis of the vessel, thrombosis, occlusion, or an aneurysm.

One type of endoluminal prosthesis used in the repair of diseases in various body vessels is a stent. A stent is a generally longitudinal tubular device formed of biocompatible material which is useful to open and support various lumens in the body. For example, stents may be used in the vascular system, urogenital tract and bile duct, as well as in a variety of other applications in the body. Endovascular stents have become widely used for the treatment of stenosis, strictures, and aneurysms in various blood vessels. These devices are implanted within the vessel to open and/or reinforce collapsing or partially occluded sections of the vessel.

Stents are generally open ended and are radially expandable between a generally unexpanded insertion diameter and an expanded implantation diameter which is greater than the unexpanded insertion diameter. Stents are often flexible in configuration, which allows them to be inserted through and conform to tortuous pathways in the blood vessel. The stent is generally inserted in a radially compressed state and expanded either through a self-expanding mechanism, or through the use of balloon catheters.

A graft is another type of endoluminal prosthesis which is used to repair and replace various body vessels. Whereas a stent provides structural support to hold a damaged vessel open, a graft provides an artificial lumen through which blood may flow. Grafts are tubular devices which may be formed of a variety of material, including textiles, and non-textile materials. One type of non-textile material particularly suitable for use as an implantable prosthesis is polytetrafluoroethylene (PTFE). PTFE exhibits superior biocompatibility and low thrombogenicity, which makes it particularly useful as vascular graft material in the repair or replacement of blood vessels. In vascular applications, the grafts are manufactured from expanded PTFE (ePTFE) tubes. These tubes have a microporous structure which allows natural tissue ingrowth and cell endothelization once implanted in the vascular system. This contributes to long term healing and patency of the graft.

It is also known to combine a stent and a graft to form a composite medical device. Such a composite medical device provides additional support for blood flow through weakened sections of a blood vessel. In endovascular applications the use of a stent/graft combination is becoming increasingly important because the combination not only effectively allows the passage of blood therethrough, but also ensures the implant will remain open.

Several types of stent/graft inventions are known in the art. U.S. Patent No. 5,151,105 issued to Kwan-Gett discloses a collapsible textile vessel sleeve with stent members positioned at opposite ends of the sleeve. The device is specifically designed to provide a vessel sleeve that is collapsible to a very small diameter in order that it may be placed in position within the abdominal or thoracic aorta by a catheter via the lumen of the femoral artery. Such a procedure obviates the need for a major surgical intervention, and reduces the risks associated with such a procedure.

Other stent/graft composite devices using a textile fabric are shown in U.S. Patent No. 5,628,788, to Pinchuck.

As mentioned above, ePTFE may also be used as graft material in stent/graft endoprosthesis. One example of an ePTFE stent/graft composite device is shown in U.S. Patent No. 5,700,285 issued to Myers, et al. Myers discloses a tubular intraluminal graft in the form of a tubular diametrically adjustable stent having an interior and exterior tubular covering of porous expanded polytetrafluoroethylene. The tubular covering surrounds the stent so that the stent is contained during contraction and expansion in the delivery process.

Stents are effectively used in combination with grafts as the composite endoluminal prosthesis allows blood flow through the vessel created by the graft, while the stent maintains its patency. However, as the graft covers the stent, it has a tendency to reduce the longitudinal flexibility of the composite device. Longitudinal compliance is of particular importance to such stent/graft endoluminal prosthesis as the device must be intraluminally delivered through tortuous pathways of a blood vessel to the implantation site where the stent is expanded. A reduction in longitudinal flexibility makes intraluminal delivery more difficult. Reduction in longitudinal flexibility is particularly evident with stents covered by ePTFE, which is not as compliant or flexible as textile materials.
A composite stent-graft prosthesis according to the preamble of claim 1 is disclosed in EP-A- 0893 108.

### SUMMARY OF THE INVENTION

It is therefore an object of the present invention to provide an endoluminal prosthesis including a stent covered with a graft providing increased longitudinal flexibility.

It is a further object of the present invention to provide a composite stent/graft providing increased compliance and longitudinal flexibility.

It is a still further object of the present invention to provide a composite stent/graft having the fluid retention features of an ePTFE graft without losing the longitudinal flexibility and compliance of a stent.

In the efficient attainment of these and other objectives, the present invention provides a composite stent-graft tubular prosthesis including an inner PTFE tubular structure, and an outer PTFE tubular structure positioned about the inner PTFE tubular structure. A diametrically deformable stent is interposed between the inner and outer PTFE tubular structure. The interposed stent is formed from an elongate wire helically wound with a plurality of longitudinally spaced turns into an open tubular configuration. Each of the turns include successive upper and lower wave-like peaks wherein selective ones of said upper and lower peaks are exposed exteriorly of the outer PTFE structure. The outer PTFE tubular structure indudes a plurality of apertures there through and the selective upper and lower peaks are aligned with the aperture.

The present invention may further embody a composite stent-graft tubular prosthesis comprising a first PTFE tubular structure with a diametrically deformable stent positioned over said first PTFE tubular structure. The stent being formed of an elongate helically wound wire formed into an open tubular configuration by a plurality of turns. The helically wound wire includes a plurality of transverse generally wave-like undulations there along defining successive upper and lower peaks. The tubular prosthesis further includes a second PTFE tubular structure positioned over said stent. The second PTFE tubular structure includes a plurality of apertures therethrough, the apertures being aligned with selective ones of said upper and lower peaks of the stent to expose said upper and lower peaks to thereby enhance longitudinal flexibility of said prosthesis.

A method of making a stent-graft luminal prosthesis of the present invention is also disclosed. The method provides for the formation of a first PTFE tubular structure. A stent is positioned over said first PTFE tubular structure, the stent having a tubular configuration formed of a plurality of turns of a helically wound wire, with each of said turns including successive upper and lower wave-like peaks. A second PTFE tubular structure is then formed over said stent, with said second PTFE tubular structure exposing selective ones of said upper and lower wave-like peaks through said second PTFE tubular structure. The exposing step indudes: forming the second PTFE tubular structure with a plurality of apertures. the apertures being at a location adjacent the selective upper and lower peaks.

### BRIEF DESCRIPTION OF THE DRAWINGS

Figure 1 is a perspective showing, partially in section of a portion of an endolmninal prosthesis of the present invention.
Figure 2 shows a longitudinal cross-section of the endoluminal prosthesis of Figure 1 taken through the lines 2-2 thereof.
Figure 3 shows a longitudinal cross-section of the endoluminal prosthesis of Figure 1 in a flexed position taken through the line 3-3 thereof.
Figure 4 shows an enlarged cross-section of a portion of the flexed prosthesis of the present invention.
Figure 5 is a perspective of a further embodiment of the endolmninal prosthesis of the present invention.
Figure 6 is a perspective of a stent which may be used in the endoluminal prosthesis of the present invention.

### DETAILED DESCRIPTION OF THE PREFERRED EMBODIMENTS:

The following is a detailed description of the preferred embodiments of the present invention. The description is meant to describe the preferred embodiments, and is not meant to limit the invention in any way.

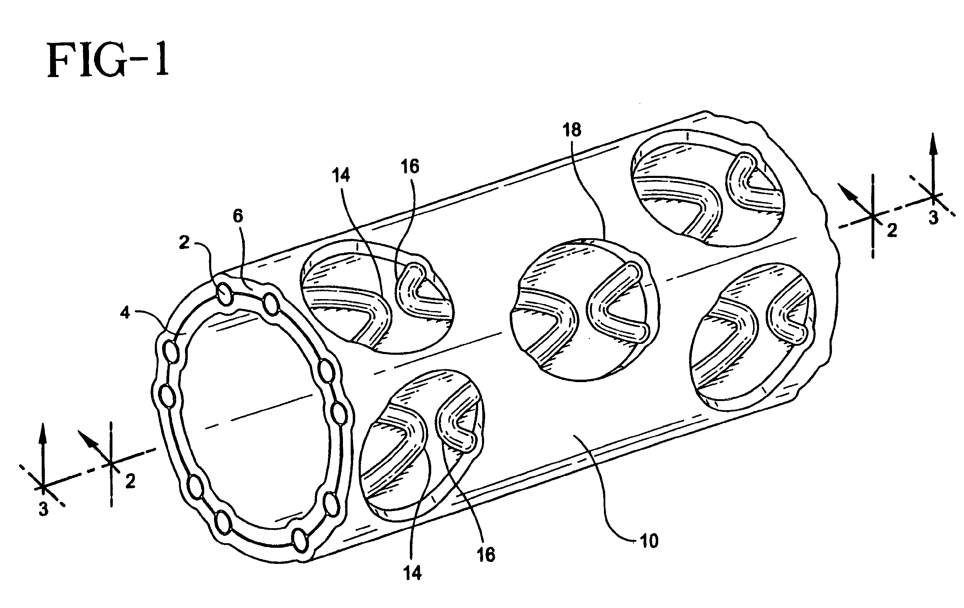

A stent/graft prosthesis 10 of the present invention is shown in Figure 1. The prosthesis includes a stent 2, an inner tubular layer 4, and an outer tubular layer 6. The stent is positioned between the inner and outer tubular layers.

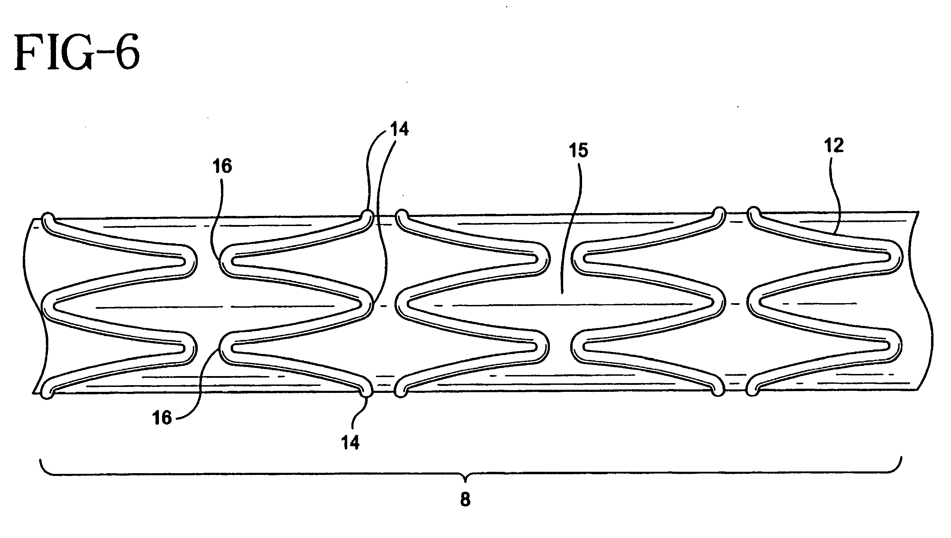

The stent in the present invention is formed from an elongate wire 12 which is helically wound with a plurality of longitudinally spaced turns into an open tubular configuration. As partially shown in Figure 6, a stent 2 is of the type which is particularly suited for use in the endoluminal prosthesis of the present invention. The stent 2 is an expandable tubular member which may be either of the balloon-expanded or self-expanded type. Stents of this type are typically introduced intraluminally into the body, and expanded at the implantation site. The elongate helically wound wire 12 forming stent 2, defines successive upper wave-like peaks 14, and lower wave-like peaks 16. The wire 12 is wound into a specific configuration where upper peaks 14 are placed adjacent to lower peaks 16 of the next adjacent winding. While the specific configuration of the stent 2 shown herein has been found to be preferable, other stent configurations having open cell expandable construction are within the contemplation of the present invention.

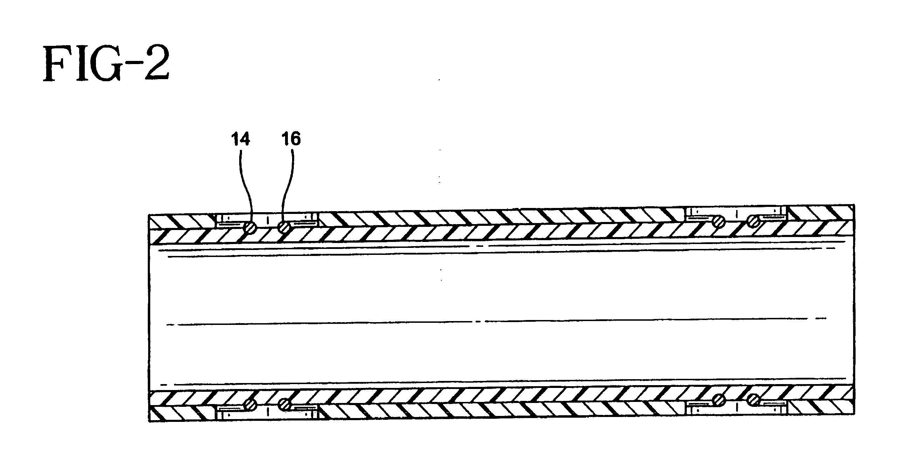

Referring now to Figures 1 and 2, inner and outer tubular layers, 4 and 6 respectively, are shown surrounding stent 2. Layers 4 and 6 are formed of extruded polytetrafluoroethylene (PTFE), as extruded PTFE exhibits superior biocompatibility. Further, PTFE is particularly suitable for vascular applications as it exhibits low thrombogenicity. Tubes of extruded PTFE may also be expanded to form ePTFE tubes. The advantages of the use of ePTFE materials in forming tubular structures for use as vascular grafts are well known.

PTFE may be extruded as a tube or may be extruded as a sheet or film, and subsequently wrapped around a mandrel to form a tubular structure. While ePTFE tubular structures, whether tubes or wrapped ePTFE sheets, exhibit advantageous biophysical compatibility qualities, such structures have a tendency when used in combination with an open stent to reduce the flexibility and longitudinal compliance of the device. This is especially evident where the tubular stent is covered either internally and/or externally with such tubular structures. The present invention is intended to create a stent-graft endoluminal prosthesis exhibiting the beneficial physical properties of ePTFE grafts, without significantly reducing the flexibility of the expandable stent.

In order to increase longitudinal flexibility of stent-graft prosthesis 10, outer PTFE tubular layer 6 is constructed so that it exposes exteriorly the upper wave-like peaks 14, and lower wave-like peaks 16 of stent 2. As mentioned above in a preferred embodiment, the upper wave-like peaks 14 and lower wave-like peaks 16 of the stent are aligned in juxtaposition at various locations along the stent. At such locations the outer tubular layer 6 may be found to have a plurality of apertures 18. These apertures 18 expose the aligned peaks 14 and 16 exteriorly. The inner layer 4 remains continuous particularly underlying apertures 18 so as to maintain a solid tube which functions as a graft.

In the presently described embodiment, all of the stent peaks of a given stent in the prosthesis are exposed. It is contemplated further that only a selected number of aligned peaks may be exposed exteriorly of the endoluminal prosthesis.

The apertures 18 which expose the peaks may be formed into a variety of shapes. As particularly shown in Figure 1, the apertures 18 are formed as circular holes through which the successive peaks 14 and 16 are exposed. However other aperture configurations may also be employed.

The stent/graft composite device of the present invention is constructed by initially forming a first inner tubular layer 4. As mentioned above, tubular layer 4 may be formed from an extruded tube or a formed extruded sheet by processes well known in the art. Stent 2 is then positioned over the inner PTFE tubular layer 4. Stent 2 has a tubular configuration formed of a plurality of turns of a helically wound wire, each of said turns including successive upper and lower wave-like peaks. The wound wire is formed such that peaks 14 and 16 are placed in aligned juxtaposition. A second outer PTFE tubular layer 6 is then formed over stent 2. The second outer tubular layer is then modified so as to expose selective aligned upper and lower wave-like peaks through the second PTFE tubular layer 6.

In a preferred embodiment of the present invention, the stent/graft prosthesis includes an open wall stent 12 of tubular construction supported between an inner PTFE tubular layer 4, and an outer PTFE tubular layer 6. The inner PTFE tubular layer 4 may be bonded to the outer PTFE tubular layer through the spaces 15 in the open wall of the stent 2. The bonding may be effectuated with the use of an adhesive, or by adhering the layers together without an adhesive. Bonding of the PTFE layers without an adhesive may take place by such methods as laminating, or sintering of the prosthesis. Furthermore, the stent may be adhered to the inner PTFE tubular layer, the outer PTFE tubular layer, or both. Similarly, such adherence may take place with or without the use of an adhesive.

Once the stent 2 is positioned between layers 4 and 6, a cutting tool (not shown) may be used to expose upper and lower wave-like peaks of the stent exteriorly of the outer PTFE tubular structure. The cutting tool may be used to cut apertures in the outer tubular layer 6 so as to expose the peaks of the stent. Some cutting tools which may be used include a razor blade and a laser. The prosthesis may be sintered prior to exposing the stent peaks, or after said exposure step.

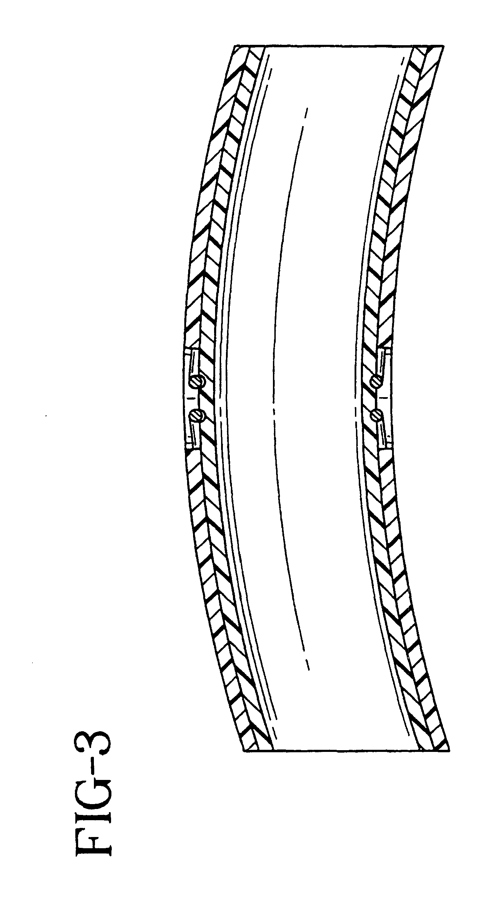

The increased longitudinal flexibility and compliance of the prosthesis may be clearly illustrated with reference to Figures 2-4.

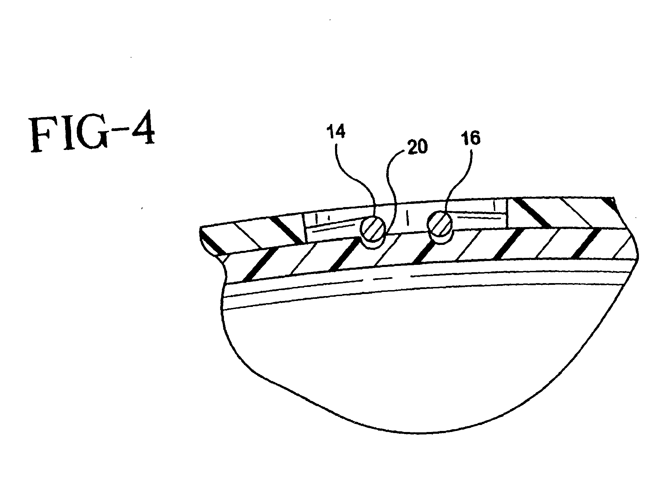

As described above the successive upper peaks 14 are juxtaposed with respect to the lower peaks 16. The juxtapositioning of the successive peaks creates a flexion similar to a joint. To further optimize flexibility, the endoluminal prosthesis reduces any movement restriction as between peaks 14 and 16, as the juxtaposed peaks of the stent are exposed exteriorly of the outer PTFE structure. Figure 4 shows such a longitudinal flexion in the endoluminal prosthesis. Such longitudinal flexion may be the result of intraluminal delivery of the device through the tortuous blood vessel system. As the juxtaposed peaks are exposed exteriorly of the outer PTFE structure, the exposed peaks have a tendency to project extemally away from the first tubular structure, creating a space 20 between the stent and the inner tubular structure when longitudinally flexed. The exposure of the peaks enables the stent to exhibit uncovered flexibility as the stent is not restricted from exhibiting movement between the wire windings as would be the case if the stent were completely covered.

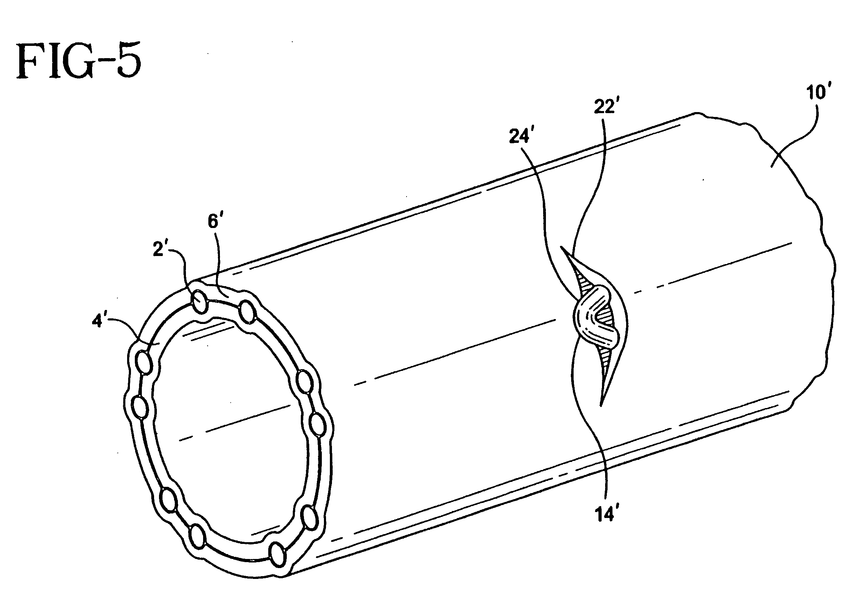

As set forth in the above preferred embodiment, longitudinal compliance of the device may be achieved by placing apertures in the outer layer over the aligned peaks of the stent. However, other techniques may be employed to increase the longitudinal compliance of the covered stent. Referring now to Figure 5, a composite stent/graft prosthesis 10' is shown. Prosthesis 10' is substantially similar to stent/graft prosthesis 10 of Figure 1, having a stent 2', an inner tubular layer 4' and an outer tubular layer 6'. The stent peaks 14 and 16 may be exteriorly exposed to increase the longitudinal flexibility of the prosthesis 10'.

In the embodiment of Figure 5, the peaks 14' may be exposed by accessing the stent 2' through the outer layer 6'. A cutting tool (not shown) may be used to cut slits 22' in the outer tubular structure through which the stent peaks 14' are exposed. The stent peaks are subsequently pulled through said slits, and the PTFE comprising the outer tubular structure is subsequently tucked underneath the stent peaks at area 24'. This embodiment provides flexibility in a similar manner because the stent peaks are similarly exposed and the construction allows a full range of motion of the stent peaks. While exposure of only one peak 14' is shown in Figure 5, it may be appreciated that the exposure of other peaks 14' and 16' may be provided.

## Claims

1. A composite stent-graft tubular prosthesis (10) comprising:
an inner PTFE tubular structure (4);
an outer PTFE tubular structure (6) positioned about said inner PTFE tubular structure; and
a diametrically deformable stent (2) interposed between said inner and outer PTFE tubular structure, said stent being formed from an elongate wire (12) helically wound with a plurality of longitudinally spaced turns into an open tubular configuration, each of said turns including successive upper (14) and lower (16) wave-like peaks charaterized in that selective ones of said upper and lower peaks are exposed exteriorly of said outer PTFE structure
wherein said outer PTFE tubular structure includes a plurality of apertures (18) therethrough and wherein said selective upper and lower peaks are aligned with said apertures.

2. A composite stent-graft prosthesis of claim 1 wherein said apertures
are slits, said selective upper and lower peaks extending through said slits.

3. A composite stent-graft prosthesis of any of the preceding claims, wherein said upper peaks of one turn are juxtaposed with respect to said lower peaks of an adjacent turn.

4. A composite stent-gaft prosthesis of claim 3 wherein each aperture of said outer tubular structure exposes said juxtaposed upper and lower peaks.

5. A composite stent-graft prosthesis of any of the preceding claims, wherein said inner and outer PTFE tubular structures are formed of sheets.

6. A composite stent-graft prosthesis of any of the preceding claims, wherein said outer PTFE tubular stent is adheringly secured to said inner PTFE tubular structure at spaces (15) between said wound wire.

7. A composite stent-graft prosthesis of any of the preceding claims, wherein said outer PTFE tubular structure is laminated to said inner PTFE tubular stent.

8. A composite stent-graft prosthesis of any of the preceding claims said helically wound wire including a plurality of transverse generally wave-like undulations therealong defining said successive upper and lower peaks; and
said outer PTFE tubular structure including a plurality of apertures therethrough, said apertures being aligned with selective ones of said upper and lower peaks to expose said upper and lower peaks to thereby enhance longitudinal flexibility of said prosthesis.

9. A composite stent-graft prosthesis of claim 8 wherein said stent includes said wave-like undulations being arranged such that the upper peaks of one turn are juxtaposed with the lower peak of an adjacent turn.

10. A composite stent-graft prosthesis of claim 9 wherein said aperture of said second PTFE tubular structure are aligned with selective ones of said juxtaposed upper and lower peaks.

11. A composite stent-gaft prosthesis of any of the claims 8-10, wherein said first and second tubular structures are formed of expanded PTFE.

12. A composite stent-graft prosthesis of any of the claims 8-10, wherein said first and second tubular structures are formed from PTFE sheets.

13. A composite stent-graft prosthesis of any of the claims 8-12, wherein said first PTFE tubular structure is laminated to said second PTFE tubular structure through said wound wire

14. A method of forming a stent-graft prosthesis (10) comprising the step of
forming a first PTFE tubular structure (4),
positioning a stent (2) over said first PTFE tubular structure, said stent having a tubular configuration formed of a plurality of turns of a helically wound wire, each of said turns including successive upper (14) and lower (16) wave-like peaks;
forming a second PTFE tubular structure (6) over said stent; and
**characterized by** exposing selective ones of said upper and lower wave-like peaks through said second PTFE tubular structure, said exposing step including: forming said second PTFE tubular structure with a plurality of apertures, said apertures being at a location adjacent said selective upper and lower peaks.

15. A method of claim 14 further including the step of adheringly securing said first PTFE tubular structure to said second PTFE tubular structure through spaces between said wound wire.

16. A method of claim 14 or 15 wherein said apertures are slits through said second PTFE tubular structure at a location aligned with said selective upper and lower peaks of said stent; and wherein said exposing step includes:
extending said upper and lower peaks of said stent through said slits.

17. A method of claim 16 wherein said extending step includes:
lifting said selective upper and lower peaks;
tucking a portion of said second PTFE tubular structure adjacent said slit, under said upper and lower peaks.

18. A method of any of the claims 16-17, wherein said placing step includes:
cutting said slits using a cutting tool.

19. A method of any of the claims 16-18, wherein said placing step includes:
laser burning said slits through said second PTFE tubular structure.

20. A method of any of the claims 14-19, wherein said second PTFE tubular structure is sintered prior to said exposing step.

21. A method of any of the claims 14-19, wherein said second PTFE tubular structure is sintered subsequent to said exposing step.

## Patentansprüche

1. Röhrenförmige Stent-Graft-Verbundprothese (10) die umfasst:
einen inneren röhrenförmigen PTFE-Aufbau (4),
einen äußeren röhrenförmigen PTFE-Aufbau (6), der um den inneren röhrenförmigen PTFE-Aufbau herum angeordnet ist, und
einen im Durchmesser verformbaren Stent (2), der zwischen dem inneren und dem äußeren röhrenförmigen PTFE-Aufbau angeordnet ist,
wobei der Stent aus einem länglichen Draht (12) besteht, der spiralförmig mit mehreren, in Längsrichtung beabstandeten Windungen zu einer offenen röhrenförmigen Konfiguration gewickelt ist,
wobei jede der Windungen aufeinanderfolgend obere (14) und untere (16) wellenförmige Scheitel aufweist,
**dadurch gekennzeichnet, dass**
von den oberen und unteren Scheiteln ausgewählte Scheitel außerhalb des äußeren PTFE-Aufbaus freiliegen,
wobei der äußere röhrenförmige PTFE-Aufbau eine Vielzahl von durchgehenden Öffnungen (18) enthält und
die ausgewählten oberen und unteren Scheitel zu den Öffnungen ausgerichtet sind.

2. Stent-Graft-Verbundprothese nach Anspruch 1, bei der die Öffnungen Schlitze sind und die ausgewählten oberen und unteren Scheitel sich durch diese Schlitze erstrecken.

3. Stent-Graft-Verbundprothese nach einem der vorhergehenden Ansprüche, bei der die oberen Scheitel einer Windung in Bezug auf die oberen Scheitel einer benachbarten Windung nebeneinander liegen.

4. Stent-Graft-Verbundprothese nach Anspruch 3, bei der bei jeder Öffnung des äußeren röhrenförmigen Aufbaus die nebeneinanderliegenden unteren und oberen Scheitel freiliegen.

5. Stent-Graft-Verbundprothese nach einem der vorhergehenden Ansprüche, bei der die inneren und äußeren röhrenförmigen PTFE-Aufbauten aus Folien bestehen.

6. Stent-Graft-Verbundprothese nach einem der vorhergehenden Ansprüche, bei der der äußere röhrenförmige PTFE-Stent durch Haftung am inneren röhrenförmigen PTFE-Aufbau in den Zwischenräumen (15) zwischen dem gewickelten Draht befestigt ist.

7. Stent-Graft-Verbundprothese nach einem der vorhergehenden Ansprüche, bei welcher der äußere röhrenförmige PTFE-Aufbau an den inneren röhrenförmigen PTFE-Stent anlaminiert ist.

8. Stent-Graft-Verbundprothese nach einem der vorhergehenden Ansprüche,
bei welcher der spiralförmig gewickelte Draht eine Vielzahl von Querwelligkeiten aufweist, die im allgemeinen wellenförmig sind und die aufeinanderfolgenden oberen und unteren Scheitel vorgeben, und
der äußere röhrenförmige PTFE-Aufbau eine Vielzahl von durchgehenden Öffnungen aufweist, die mit ausgewählten oberen und unteren Scheiteln ausgerichtet sind, so dass die oberen und unteren Scheitel freiliegen und **dadurch** die Flexibilität der Prothese in Längsrichtung erhöht wird.

9. Stent-Graft-Verbundprothese nach Anspruch 8, wobei der Stent die wellenförmige Welligkeit aufweist, die so gestaltet ist, dass die oberen Scheitel einer Windung und der untere Scheitel einer benachbarten Windung nebeneinanderliegen.

10. Stent-Graft-Verbundprothese nach Anspruch 9, wobei die Öffnungen des zweiten röhrenförmigen PTFE-Aufbaus in Bezug auf ausgewählte Scheitel der nebeneinanderliegenden unteren und oberen Scheitel ausgerichtet sind.

11. Stent-Graft-Verbundprothese nach Ansprüchen 8 bis 10, wobei der erste und der zweite röhrenförmige Aufbau aus expandiertem Polytetrafluorethylen bestehen.

12. Stent-Graft-Verbundprothese nach Ansprüchen 8 bis 10, wobei der erste und der zweite röhrenförmige Aufbau aus PTFE-Folien bestehen.

13. Stent-Graft-Verbundprothese nach Ansprüchen 8 bis 12, wobei der erste röhrenförmige PTFE-Aufbau als Schicht mit Hilfe des gewickelten Drahtes auf den zweiten röhrenförmigen PTFE-Aufbau aufgebracht ist.

14. Verfahren zur Herstellung einer Stent-Graft-Verbundprothese, das folgende Schritte umfasst:
Ausbildung eines ersten röhrenförmigen PTFE-Aufbaus (4), Positionierung eines Stents (2) über dem ersten röhrenförmigen PTFE-Aufbau, wobei der Stent eine röhrenförmige Konfiguration aufweist, die aus einer Vielzahl von Windungen eines spiralförmig gewickelten Drahtes gebildet ist, wobei jede Windung aufeinanderfolgende obere (14) und untere (16) wellenförmige Scheitel enthält, und
Ausbildung eines zweiten röhrenförmigen PTFE-Aufbaus (6) über dem Stent,
**gekennzeichnet durch**
Freilegung ausgewählter Scheitel der oberen und unteren wellenartigen Scheitel **durch** den zweiten röhrenförmigen PTFE-Aufbau hindurch, wobei der Schritt der Freilegung umfasst:
Ausbildung des zweiten röhrenförmigen PTFE-Aufbaus mit einer Vielzahl von Öffnungen, wobei sich die Öffnungen an einer Stelle befinden, die nahe an den ausgewählten oberen und unteren Scheiteln liegt.

15. Verfahren nach Anspruch 4, das weiter den Schritt der Haftbefestigung des ersten röhrenförmigen PTFE-Aufbaus am zweiten röhrenförmigen PTFE-Aufbau in Abständen zwischen dem gewickelten Draht enthält.

16. Verfahren nach Anspruch 14 oder 15, bei dem die Öffnungen Schlitze im zweiten röhrenförmigen PTFE-Aufbau an Stellen sind, die mit den ausgewählten unteren und oberen Scheiteln des Stents ausgerichtet sind, und der Schritt der Freilegung die Erstreckung der oberen und unteren Scheitel des Stents durch die Schlitze hindurch umfasst.

17. Verfahren nach Anspruch 16, bei dem der Schritt der Erstreckung das Anheben der ausgewählten oberen und unteren Scheitel und das Einlegen eines Teils des zweiten röhrenförmigen PTFE-Aufbaus nahe am Schlitz unter die oberen und unteren Scheitel umfasst.

18. Verfahren nach Ansprüchen 16 und 17, bei dem der Schritt der Anordnung das Schneiden der Schlitze mit Hilfe eines Schneideinstruments umfasst.

19. Verfahren nach Ansprüchen 16 bis 18, bei dem der Schritt der Anordnung das Brennen der Schlitze durch den zweiten röhrenförmigen PTFE-Aufbau mit einem Laser umfasst.

20. Verfahren nach Ansprüchen 14 bis 19, bei dem der zweite röhrenförmige PTFE-Aufbau vor dem Schritt der Freilegung gesintert wird.

21. Verfahren nach Ansprüchen 14 bis 19, bei dem der zweite röhrenförmige PTFE-Aufbau nach dem Schritt der Freilegung gesintert wird.

## Revendications

1. Prothèse tubulaire stent - greffon composite (10) comprenant:
une structure tubulaire interne en PTFE (4) ;
une structure tubulaire externe en PTFE (6) positionnée autour de ladite structure tubulaire interne en PTFE ; et
un stent déformable diamétralement (52) interposé entre ladite structure tubulaire interne en PTFE et ladite structure tubulaire externe en PTFE, ledit stent étant formé d'un fil allongé (12) enroulé en hélice avec une pluralité d'enroulements espacés longitudinalement en une configuration tubulaire ouverte, chacun desdits enroulements comprenant des pics en forme d'onde supérieurs (14) et inférieurs (16) successifs, **caractérisée en ce que** certains desdits pics supérieurs et inférieurs sont exposés à l'extérieur de ladite structure externe en PTFE, dans laquelle ladite structure tubulaire externe en PTFE comporte une pluralité d'ouvertures (18) la traversant et dans laquelle lesdits certains pics supérieurs et inférieurs sont alignés avec lesdites ouvertures.

2. Prothèse stent - greffon composite selon la revendication 1 dans laquelle lesdites ouvertures sont des fentes, lesdits certains pics supérieurs et inférieurs s'étendant à travers lesdites fentes.

3. Prothèse stent - greffon composite selon l'une quelconque des revendications précédentes, dans laquelle lesdits pics supérieurs d'un enroulement sont juxtaposés auxdits pics inférieurs d'un enroulement adjacent.

4. Prothèse stent - greffon composite selon la revendication 3, dans laquelle chaque ouverture de ladite structure tubulaire externe expose lesdits pics supérieurs et inférieurs juxtaposés.

5. Prothèse stent - greffon composite selon l'une quelconque des revendications précédentes, dans laquelle ladite structure tubulaire interne en PTFE et ladite structure tubulaire externe en PTFE sont formées de feuillets.

6. Prothèse stent - greffon composite selon l'une quelconque des revendications précédentes, dans laquelle ledit stent tubulaire externe en PTFE est fixé par collage à ladite structure tubulaire interne en PTFE en des espaces (15) entre ledit fil enroulé.

7. Prothèse stent - greffon composite selon l'une quelconque des revendications précédentes, dans laquelle ladite structure tubulaire externe en PTFE est laminée au dit stent tubulaire interne en PTFE.

8. Prothèse stent - greffon composite selon l'une quelconque des revendications précédentes, ledit fil enroulé en hélice comportant une pluralité d'ondulations en forme d'onde généralement transversales sur leurs longueurs définissant lesdits pics supérieurs et inférieurs successifs ; et
ladite structure tubulaire externe en PTFE comportant une pluralité d'ouvertures la traversant, lesdites ouvertures étant alignées avec certains desdits pics supérieurs et inférieurs pour exposer lesdits pics supérieurs et inférieurs pour ainsi améliorer la flexibilité longitudinale de ladite prothèse.

9. Prothèse stent - greffon composite selon la revendication 8, dans laquelle ledit stent comporte lesdites ondulations en forme d'onde étant arrangées de telle sorte que les pics supérieurs d'un enroulement soient juxtaposés au pic inférieur d'un enroulement adjacent.

10. Prothèse stent - greffon composite selon la revendication 9, dans laquelle ladite ouverture de ladite seconde structure tubulaire en PTFE est alignée avec certains desdits pics supérieurs et inférieurs juxtaposés.

11. Prothèse stent - greffon composite selon l'une quelconque des revendications 8 à 10, dans laquelle ladite première structure tubulaire et ladite seconde structure tubulaire sont formées de PTFE expansé.

12. Prothèse stent - greffon composite selon l'une quelconque des revendications 8 à 10, dans laquelle ladite première structure tubulaire en PTFE et ladite seconde structure tubulaire sont formées de feuilles de PTFE.

13. Prothèse stent - greffon composite selon l'une quelconque des revendications 8 à 12, dans laquelle ladite première structure tubulaire est laminée à ladite seconde structuré tubulaire en PTFE par ledit fil enroulé.

14. Procédé pour former une prothèse stent - greffon composite (10) comprenant les étapes de :
formation d'une première structure tubulaire en PTFE (4) ;
positionnement d'un stent (2) sur ladite première structure tubulaire en PTFE, ledit stent ayant une configuration tubulaire formée d'une pluralité d'enroulements d'un fil enroulé en hélice, chacun desdits enroulements comportant des pics en forme d'onde supérieurs (14) et inférieurs (16) successifs ;
formation d'une seconde structure tubulaire en PTFE (6) sur ledit stent ; et
**caractérisé par** l'exposition de certains desdits pics en forme d'onde supérieurs et inférieurs à travers ladite seconde structure tubulaire en PTFE, ladite étape une exposition comportant : une formation de ladite seconde structure tubulaire en PTFE avec une pluralité d'ouvertures, lesdites ouvertures étant en un emplacement adjacent auxdits certains pics supérieurs et inférieurs.

15. Procédé selon la revendication 14 comportant en outre une étape de fixation par collage de ladite première structure tubulaire en PTFE à ladite seconde structure tubulaire en PTFE à travers des espaces entre ledit fil enroulé.

16. Procédé selon la revendication 14 ou la revendication 15, dans lequel lesdites ouvertures sont des fentes traversant ladite seconde structure tubulaire en PTFE en un emplacement aligné avec lesdits certains pics supérieurs et inférieurs dudit stent, et dans lequel ladite étape d'exposition comporte une extension desdits pics supérieurs et inférieurs dudit stent à travers lesdites fentes.

17. Procédé selon la revendication 16, dans lequel ladite étape d'extension comporte :
un soulèvement desdits certains pics supérieurs et inférieurs ;
une mise en place d'une partie de ladite seconde structure tubulaire en PTFE adjacente à ladite fente, sous lesdits pics supérieurs et inférieurs.

18. Procédé selon l'une quelconque des revendications 16 à 17, dans lequel ladite étape de mise en place comporte :
une découpe desdites fentes avec un outil de découpe.

19. Procédé selon l'une quelconque des revendications 16 à 18, dans lequel ladite étape de mise en place comporte :
une brûlure au laser desdites fentes à travers ladite seconde structure tubulaire en PTFE.

20. Procédé selon l'une quelconque des revendications 14 à 19, dans lequel ladite seconde structure tubulaire en PTFE est frittée avant ladite étape d'exposition.

21. Procédé selon l'une quelconque des revendications 14 à 19, dans lequel ladite seconde structure tubulaire en PTFE est frittée après ladite étape d'exposition.
